# FASCICULE DE BREVET EUROPEEN

(11) **EP 0 685 450 B1**
(45) Date de publication et mention de la délivrance du brevet: **14.07.1999**
(21) Numéro de dépôt: 95106438.5
(22) Date de dépôt: 28.04.1995
(51) Int. Cl.: C07C 67/055, C07C 69/013

(54) **Procédé pour la préparation d'esters carboxyliques dérivés d'alcools allyliques**
Verfahren zum Herstellen von Allylalkohol abgeleiteten Carbonsäureestern
Process for the preparation of esters of carboxylic acids derived from allylic alcohol

(30) Priorité: 01.06.1994 CH 170494
(43) Date de publication de la demande: 06.12.1995
(73) Titulaire: FIRMENICH SA, 1211 Genève 8 (CH)
(72) Inventeur: Mimoun, Hubert, F-01630 Challex (FR); Jia, Chengguo, Ames, Iowa 50011 (US)
(74) Mandataire: Salvaterra-Garcia, Maria de Lurdes

(56) Documents cités:
- DE-B- 1 191 362

## Description

Nombreux sont les esters carboxyliques dérivés d'alcools allyliques mentionnés dans la littérature à titre d'intermédiaires utiles pour la préparation de composés d'intérêt pour l'industrie de la parfumerie et des arômes, ainsi que pour différentes spécialités de l'industrie pharmaceutique.

Or, de tels composés ont été obtenus par le passé, avec un degré mitigé de succès, par oxydation des oléfines correspondantes.

Différentes méthodes ont été élaborées à cet effet. Aucune toutefois n'est parfaitement satisfaisante.

Lorsque l'oxydation de ces oléfines est effectuée par exemple par autoxydation à l'air, elle donne lieu à des réactions peu sélectives, l'hydroperoxide primaire intermédiaire se décomposant pour fournir un mélange d'énols et d'énones. Une telle réaction requiert d'autre part des réacteurs de grand volume et un recyclage de l'oléfine de départ.

Une autre méthode qui consiste en l'oxydation allylique des oléfines par l'action du dioxyde de sélénium est également peu sélective et requiert l'emploi de quantités importantes de SeO₂, réactif toxique et nuisible pour l'environnement.

Une autre méthode connue d'oxydation allylique a recours à l'emploi de catalyseurs au palladium. L'acétate d'allyle, intermédiaire utile pour la préparation d'alcool allylique, peut être obtenu, par exemple, par acétoxylation du propène en présence de palladium. Il en va de même de la fabrication industrielle du 1,4-diacétoxy-but-2-ène.

En général, dans ce type de réaction, on emploie un catalyseur au palladium, de préférence sur un support solide, tel par exemple la silice ou l'alumine dopé par des acétates alcalins et/ou des sels d'or, de fer ou de bismuth (acétate d'allyle) ou par l'oxyde de tellure (1,4-diacétoxy-butène) [voir H. Mimoun dans "Comprehensive Coordination Chemistry", Ed. Wilkinson, vol. 6, p. 318 (1983) et références incluses]. Ce type de procédé s'est également révélé peu satisfaisant, car en pratique la conversion ne procède pas jusqu'à son terme.

Cet état de fait est encore plus prononcé lorsqu'on emploie comme substrat des oléfines supérieures.

Tous les systèmes décrits plus haut utilisent l'oxygène moléculaire comme agent oxydant. Lorsqu'on emploie comme catalyseur un sel du palladium, par exemple sous forme d'acétate, ce dernier est utilisé à des concentrations relativement élevées, généralement supérieures à 2% en poids par rapport au produit de départ. La réaction s'effectue également en présence de divers agents réoxydants du palladium, tels par exemple les nitrates alcalins [Tetrah. Lett. 1993, 34, 2523], ou la benzoquinone [J. Am. Chem. Soc. 1990, 112, 5160 ; Acta Chem. Scand. 1993, 47, 506 ; J. Org. Chem. 1991, 56, 5808] accompagnée de dioxyde de manganèse en concentration stoéchiométrique [J. Org. Chem 1990, 55, 975], ou encore de co-catalyseurs permettant de réoxyder l'hydroquinone formée en benzoquinone par l'oxygène moléculaire.

Ces méthodes de l'art antérieur ont recours, comme nous l'avons vu, à des systèmes redox complexes, onéreux et peu rentables pour lesquels la récupération et le recyclage du métal précieux posent des problèmes techniques difficiles.

Nous avons maintenant découvert qu'il était désormais possible d'effectuer des réactions d'acyloxylation allylique d'oléfines par réaction de ces dernières avec du peroxyde d'hydrogène en présence d'un catalyseur constitué par un sel ou un complexe du palladium et en présence d'un acide carboxylique.

La présente invention a donc pour objet un procédé pour la préparation d'esters carboxyliques dérivés d'alcools allyliques lequel procédé est caractérisé par la réaction d'une oléfine aliphatique ou cycloaliphatique comportant de 4 à 16 atomes de carbone dans sa chaîne principale et possédant une double liaison, non-terminale, avec un acide carboxylique en présence de peroxyde d'hydrogène et d'un catalyseur constitué par un sel ou un complexe de palladium.

La réaction qui caractérise le procédé de l'invention peut être illustrée à l'aide du schéma réactionnel que voici : 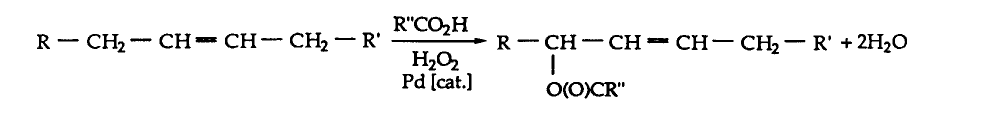 dans lequel R, R' et R'' représentent chacun un atome d'hydrogène ou un reste alkyle.

Lorsque la réaction est appliquée sur des oléfines cycloaliphatiques, ce même type de réaction permet de manière analogue l'obtention d'esters allyliques correspondants. Par exemple, par l'acyloxylation allylique de cyclopentène, on obtient le 3-acyloxy-cyclopentène

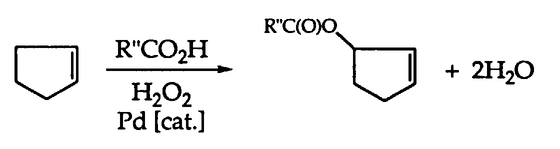

A titre d'oléfine de départ, on pourra utiliser toute une série d'oléfines mono ou polyinsaturées, à chaîne linéaire ou ramifiée, substituée ou non. Parmi celles-ci il convient de citer le cis- ou trans-but-2-ène, le 2-méthyl-but-2-ène, le pent-2-ène, le pent-3-ène, le oct-2-ène, le oct-3-ène, le oct-4-ène, le hexa-1,5-diène, le 2,4,4-triméthyl-pent-2-ène, le 1-phénylpropène, l'isosafrole ou l'iso-méthyleugénol.

Parmi les cyclooléfines on peut mentionner à titre d'exemple le cyclopentène, le cyclohexène, le cycloheptène, le cis- ou trans-cyclooctène, le cis- ou trans-cyclododécène, le cyclohexadécène, le cyclopentadiène, le cyclohexa-1,3-diène, le cyclohexa-1,4-diène, le dicyclopentadiène, le cycloocta-1,5-diène, l'α-pinène, le β-pinène, le δ-3-carène, le 4-vinyl-cyclohexène, le diméthyl-octa-1,5-diène. Nous avons observé que la réaction procède de la manière la plus sélective sur un substrat tel le cyclopentène, le cyclohexène ou le cycloheptène.

A titre d'acide carboxylique on peut utiliser une grande variété d'acides carboxyliques, possédant aussi bien une chaîne aliphatique, saturée ou insaturée, substituée ou non, qu'aromatique. A titre d'exemple on peut citer l'acide acétique, l'acide propionique, l'acide butyrique, l'acide isobutyrique, l'acide benzoïque, l'acide 2-éthyl-hexanoïque, l'acide acrylique, l'acide crotonique et l'acide méthacrylique. L'acide acétique est utilisé de préférence.

Le réactif acide est employé à raison d'au moins un équivalent molaire par rapport à l'oléfine de départ.

Comme indiqué plus haut, la réaction est effectuée en présence de peroxyde d'hydrogène. A cet effet on peut employer une solution aqueuse de H₂O₂ dont les concentrations peuvent varier dans une gamme de valeurs assez étendue. De préférence, on utilise le H₂O₂ à des concentrations de l'ordre de 30 à 70% en poids. Pour ce réactif également on en utilise au moins un équivalent molaire par rapport à l'oléfine de départ, ce qui entraîne sa conversion presque complète.

Selon un mode préférentiel du procédé de l'invention le H₂O₂ est ajouté au mélange réactionnel contenant l'oléfine, le catalyseur et l'acide carboxylique, choisis de sorte à éviter la décomposition du catalyseur et la précipitation qui en suivrait du palladium métallique.

Comme indiqué plus haut, la réaction a lieu en présence d'un catalyseur constitué par un sel ou un complexe du palladium. Nous avons observé que de tels sels étaient constitués de préférence par l'acétate ou le trifluoroacétate de palladium, le chlorure ou le bromure de palladium ou le Na₂PdCl₄. A titre de complexe du palladium, on peut utiliser de préférence le palladium tétrakis-(triphénylphosphine). On peut également utiliser un sel ou complexe de palladium déposé sur un support tel que la silice, l'alumine ou le charbon actif.

Pour des raisons économiques et pratiques on utilisera l'acétate de palladium.

Bien que l'emploi d'un co-catalyseur ne soit pas indispensable au bon déroulement de la réaction, il est apparu que la stabilité du catalyseur pouvait être améliorée en ajoutant au mélange réactionnel de la quinone. La sélectivité de la réaction en était également accrue dans la mesure où l'acyloxylation allylique était favorisée par rapport à l'époxydation des oléfines, réaction secondaire non désirée.

A titre de quinone, on utilise la benzoquinone ou la naphtoquinone, lesquels composés sont utilisés à une concentration comprise entre 1 et 10% molaires par rapport à l'oléfine de départ.

Nous avons enfin observé qu'il était avantageux d'ajouter au milieu réactionnel, en plus du catalyseur et, éventuellement, de la quinone, un support solide organique ou inorganique tel le charbon actif, la silice ou l'alumine. De tels supports favorisent non seulement la sélectivité de la réaction mais également la récupération du catalyseur et, par voie de conséquence, son recyclage.

L'invention est illustrée par les exemples suivants dans lesquels les températures sont indiquées en degrés centigrades et les abréviations ont le sens usuel dans l'art.

### Exemple 1

### Acétoxylation du cyclohexène

Dans un ballon tricol de 500 ml, on introduit 15 g de cyclohexène (0,18 moles), 150 g d'acide acétique et 80 mg d'acétate de palladium (0,36 mmoles). On porte la température à 50°, puis on introduit en 8 h 10,6 g (0,22 moles) de peroxyde d'hydrogène à 70% en poids, et on laisse réagir 2 h supplémentaires en maintenant la température à 50°. On distille ensuite l'acide acétique (35°, 20 x 10² Pa). Le résidu est alors distillé sous vide (64-68°, 15 x 10² Pa) pour donner l'acétate de cyclohex-2-én-1-yle à 90% de pureté (13 g, rend. 50%).

### acétate de cyclohex-2-én-1-yle

- RMN(¹H, 200MHz, CDCl₃) :: 5,90(m, 1H); 5,65(m, 1H); 5,21(large m, 1H); 2,02(s, 3H); 1,5-2,1(m, 6H) δppm
- RMN(¹³C):: 170,45; 132,29; 125,53; 67,84; 28,06; 24,82; 21,05; 18,80 δppm
- SM:: m/e 140(M⁺)

### Exemple 2

### Acétoxylation du cyclohexène

On opère comme décrit à l'Exemple 1, mais en ajoutant 0,77g de benzoquinone (7mmoles) et en divisant par 4 la quantité d'acétate de palladium (20 mg, 0,09 mmoles). On porte la température à 50°, puis on introduit en 8 h 10,6 g (0,22 moles) de peroxyde d'hydrogène à 70% en poids, et on laisse réagir 2 h supplémentaires en maintenant la température à 50°. On distille ensuite l'acide acétique (35°, 20 x 10² Pa). Le résidu est alors distillé sous vide (64-68°, 15 x 10² Pa) pour donner l'acétate de cyclohex-2-én-1-yle à 98% de pureté (18 g, rend. 70%).

Cet exemple montre qu'en opérant en présence de benzoquinone on augmente le rendement et la sélectivité de la réaction en utilisant des quantités nettement plus faibles de catalyseur au palladium.

### Exemple 3

### Acétoxylation du cyclopentène

Dans un ballon tricol de 500 ml, on introduit 15 de cyclopentène (0,22 moles), 150 g d'acide acétique, 0,77 g de benzoquinone (7 mmoles) et 80 mg d'acétate de palladium (0,35 mmoles). On porte la température à 50°, puis on introduit en 8 h 12 g (0,25 moles) de peroxyde d'hydrogène à 70% en poids, et on laisse réagir 2 h supplémentaires en maintenant la température à 50°. On distille ensuite l'acide acétique (35°, 20 x 10² Pa). Le résidu est alors distillé sous vide (56-60°, 15 x 10² Pa) pour donner l'acétate de cyclopent-2-én-1-yle (13 g, rend. 60% ).

### acétate de cyclopent-2-én-1-yle

- RMN(¹H, 200MHz, CDCl₃):: 6,00(m, 1H); 5,65(m,1H); 5,65(m, 1H) 1,90(s, 3H); 1,7-2,5(m, 4H) δppm
- RMN(¹³C):: 170,85; 137,36; 129,18; 80,33; 30,94; 29,64 21,14 δppm
- SM:: m/e 126(M⁺)

### Exemple 4

### Acétoxylation du cycloheptène

Dans un ballon tricol de 500 ml, on introduit 15 de cycloheptène (0,15 moles), 150 g d'acide acétique, 0,77 g de benzoquinone (7 mmoles), 1 g de charbon actif et 80 mg d'acétate de palladium (0,35 mmoles). On porte la température à 50°, puis on introduit en 8 8 g (0,17 moles) de peroxyde d'hydrogène à 70% en poids, et on laisse réagir 2h supplémentaires en maintenant la température à 50°. On distille ensuite l'acide acétique (35°, 20 x 10² Pa). Le résidu est alors distillé sous vide (75-80°, 15 x 10² Pa) pour donner l'acétate de cyclohept-2-én-1-yle (14 g, rend. 62%).

### acétate de cyclopent-2-én-1-yle

- RMN(¹H, 200MHz, CDCl₃):: 5,74(m, 1H) 5,61(m, 1H); 5,34(m, 1H); 2,00(s, 3H); 1,20-2,20(m, 8H) δppm
- RMN(¹³C):: 170,29; 133,60; 131,43; 74,29; 32,75; 28,38; 26,53; 26,48; 21,20 δppm
- SM:: m/e 154(M⁺)

### Exemple 5

### Acétoxylation du 1-méthylcyclohexène

Dans un ballon tricol de 500 ml, on introduit 15 g de 1-méthylcyclohexène (0,15 moles), 150 g d'acide acétique, 0,77 g de benzoquinone (7 mmoles), 1 g de charbon actif et 80 mg d'acétate de palladium (0,35 mmoles). On porte la température à 50°, puis on introduit en 8 h 8 g (0,17 moles) de peroxyde d'hydrogène à 70% en poids, et on laisse réagir 2h supplémentaires en maintenant la température à 50°. On distille ensuite l'acide acétique (35°, 20 x 10² Pa). Le résidu est alors distillé sous vide (75-80°, 15 x 10² Pa) pour donner l'acétate de 2-méthyl-cyclohex-2-én-1-yle (11 g, rend. 50%).

### acétate de 2-méthyl-cyclohex-2-én-1-yl

- RMN(¹H, 200MHz, CDCl₃):: 5,72(m, 1H) 5,2(t, 1H) 2,04(s, 3H) ; 1,66(d, J=1,6Hz, 3H); 1,70-2,12(m, 6H) δppm
- RMN (¹³C) :: 170,90; 131,70; 127,8; 70,66; 28,85; 25,10; 21,22; 20,09; 18,32 δppm
- SM:: m/e 154(M⁺)

### Exemple 6

### Acétoxylation du trans-cyclododécène

On procède comme dans l'exemple précédent en utilisant 24 g de trans-cyclododécène (0,14 moles), 150 g d'acide acétique, 0,77 g de benzoquinone (7 mmoles), 1 g de charbon actif, 80 mg d'acétate de palladium (0,35 mmoles) et en introduisant 5,6 g d'H₂O₂ à 70% en poids en 10 h. Après évaporation de l'acide acétique, on recueille par distillation sous vide poussé 20 g (rend. molaire 63%) d'acétate de cyclododéc-2-én-1-yle.

### acétate de cyclododéc-2-én-1-yle

- RMN(¹H):: 5,75(m, 1H) 5,35(m, 1H); 5,14(m, 3H); 2,03(s, 3H) 1,0-2,2(m, 18H) δppm
- RMN(¹³C):: 170,28; 135,13; 129,17; 70,01; 32,07; 31,64; 25,82; 25,60; 24,93; 24,88; 24,52; 24,32; 23,69; 21,45 δppm

### Exemple 7

### Acétoxylation de l'hexa-1,5-diène

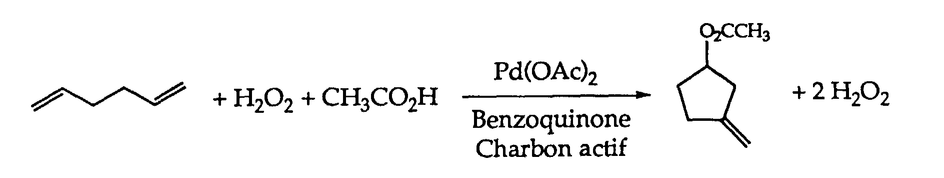

Dans un ballon tricol de 500 ml, on introduit 15 d'hexa-1,5-diène (0,18 moles), 150 g d'acide acétique, 0,77 g de benzoquinone (7 mmoles), 1 g de charbon actif et 80 mg d'acétate de palladium (0,35 mmoles). On porte la température à 50°, puis on introduit en 8h 7 g (0,14 moles) de peroxyde d'hydrogène à 70% en poids, et on laisse réagir 2h supplémentaires en maintenant la température à 50°. On distille ensuite l'acide acétique (35°, 20 x 10² Pa). Le résidu est alors distillé sous vide pour donner le 1-acétoxy-3-méthylène-cyclopentane avec 65% molaire de rendement.

### 1-acétoxy-3-méthylène-cyclopentane

- RMN(¹H):: 5,2(m, 1H); 4,91(m, 2H); 2,03(s, 3H); 2,40(m, 4H); 1,90(m, 2H) δppm
- RMN(¹³C):: 170,78; 148,83; 106,81; 75,99; 39,27; 32,17; 30,05; 21,21 δppm

## Revendications

1. Procédé pour la préparation d'esters carboxyliques dérivés d'alcools allyliques par acyloxylation allylique d'oléfines caractérisé en ce qu'on traite une oléfine aliphatique ou cycloaliphatique, comportant de 4 à 16 atomes de carbone dans sa chaîne principale et possédant une double liaison non-terminale, avec un acide carboxylique en présence de peroxyde d'hydrogène et d'un catalyseur constitué par un sel ou un complexe du palladium.

2. Procédé selon la revendication 1, caractérisé en ce que la réaction s'effectue en présence d'une quinone.

3. Procédé selon la revendication 1, caractérisé en ce que la réaction s'effectue en présence de charbon actif ou de silice.

4. Procédé selon la revendication 1, caractérisé en ce que l'acide carboxylique est employé à raison d'au moins un équivalent molaire par rapport à l'oléfine de départ.

5. Procédé selon la revendication 1, caractérisé en ce que le peroxyde d'hydrogène est utilisé à raison d'au moins un équivalent molaire par rapport à l'oléfine de départ.

6. Procédé selon la revendication 1, caractérisé en ce que l'acide carboxylique est choisi parmi les acides suivants : acétique, propionique, butyrique, benzoïque et acrylique.

7. Procédé selon la revendication 1, caractérisé en ce que le catalyseur est constitué par l'acétate, le trifluoroacétate, le chlorure ou le bromure de palladium, ou le palladium tétrakis-(triphénylphosphine).

## Claims

1. A process for the preparation of carboxylic esters derived from allylic alcohols via allylic acyloxylation of olefins, characterised in that an aliphatic or cycloaliphatic olefin, having 4 to 16 carbon atoms in its main chain and possessing a non-terminal double bond, is treated with a carboxylic acid in the presence of hydrogen peroxide and of a catalyst consisting of a palladium salt or complex.

2. A process according to claim 1, characterised in that the reaction is carried out in the presence of a quinone.

3. A process according to claim 1, characterised in that the reaction is carried out in the presence of charcoal or silica.

4. A process according to claim 1, characterised in that the carboxylic acid is used at a rate of at least one molar equivalent, with regard to the starting olefin.

5. A process according to claim 1, characterised in that the hydrogen peroxide is used at a rate of at least one molar equivalent, relative to the starting olefin.

6. A process according to claim 1, characterised in that the carboxylic acid is selected from the group consisting of acetic acid, propionic acid, butyric acid, benzoic acid and acrylic acid.

7. A process according to claim 1, characterised in that the catalyst is palladium acetate, palladium trifluoroacetate, palladium chloride or palladium bromide, or palladium tetrakis-(triphenylphosphine).

## Patentansprüche

1. Verfahren zur Herstellung von aus Allylalkoholen abgeleiteten Carbonsäureestern mittels allylischer Acyloxylierung von Olefinen, dadurch gekennzeichnet, daß ein aliphatisches oder cycloaliphatisches Olefin mit 4 bis 16 Kohlenstoffatomen in seiner Hauptkette und einer nicht-endständigen Doppelbindung in Gegenwart von Wasserstoffperoxid sowie eines aus einem Palladiumsalz oder -komplex bestehenden Katalysators mit einer Carbonsäure behandelt wird.

2. Verfahren nach Anspruch 1, dadurch gekennzeichnet, daß die Reaktion in Gegenwart eines Chinons stattfindet.

3. Verfahren nach Anspruch 1, dadurch gekennzeichnet, daß die Reaktion in Gegenwart von Aktivkohle oder Kieselsäureanhydrid stattfindet.

4. Verfahren nach Anspruch 1, dadurch gekennzeichnet, daß die Carbonsäure in einem Verhältnis von mindestens einem Moläquivalent in bezug auf das Ausgangsolefin verwendet wird.

5. Verfahren nach Anspruch 1, dadurch gekennzeichnet, daß Wasserstoffperoxid in einem Verhältnis von mindestens einem Moläquivalent in bezug auf das Ausgangsolefin verwendet wird.

6. Verfahren nach Anspruch 1, dadurch gekennzeichnet, daß die Carbonsäure unter den folgenden Säuren ausgewählt ist: Essig-, Propion-, Butter-, Benzoe- und Acrylsäure.

7. Verfahren nach Anspruch 1, dadurch gekennzeichnet, daß der Katalysator aus Palladiumacetat, -trifluoracetat, -chlorid oder -bromid oder aus Palladiumtetrakis-(triphenylphosphin) besteht.
